(19)

Europäisches
Patentamt
European
Patent Office
Office européen
des brevets

(11) **EP 2 364 695 A1**

(12) **EUROPEAN PATENT APPLICATION**

(43) Date of publication:
**14.09.2011 Bulletin 2011/37**

(51) Int Cl.:
***A61K 9/70*** *(2006.01)*     ***A61K 31/4458*** *(2006.01)*

(21) Application number: **10153194.5**

(22) Date of filing: **10.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Nitto Denko Corporation
Ibaraki-shi
Osaka 567-8680 (JP)**

(72) Inventors:
• **Hara, Takumi
Osaka 567-8680 (JP)**

• **Tamura, Kei
Osaka 567-8680 (JP)**
• **Inosaka, Keigo
Osaka 567-8680 (JP)**
• **Saeki, Yuji
Osaka 567-8680 (JP)**

(74) Representative: **von Kreisler Selting Werner
Deichmannhaus am Dom
Bahnhofsvorplatz 1
50667 Köln (DE)**

(54) **Methylphenidate patch preparation**

(57)     Provided is a methylphenidate patch preparation superior in the stability of a drug (methylphenidate and/or a salt thereof) in the patch preparation, skin permeability of a drug during use of the patch preparation, and methylphenidate availability. A patch preparation having a support and an adhesive layer formed on at least one surface of the support, wherein the adhesive layer contains methylphenidate and/or a salt thereof, polyisobutylene and a liquid plasticizer. The liquid plasticizer preferably has an HLB value of 1.0 - 3.3.

EP 2 364 695 A1

**Description**

TECHNICAL FIELD OF THE INVENTION

The present invention relates to a methylphenidate patch preparation (patch preparation containing methylphenidate and/or a salt thereof as a drug). More particularly, the present invention relates to a methylphenidate patch preparation superior in the stability of a drug in the patch preparation, skin permeability of a drug during use of the patch preparation, and drug availability.

BACKGROUND OF THE INVENTION

Attention Deficit Disorder and Attention Deficit Hyperactivity Disorder are most generally seen in children with mental disorders. The morbidity rate of Attention Deficit Disorder has been reported to be 4% - 9%.

Attention Deficit Disorder and Attention Deficit Hyperactivity Disorder are characterized by carelessness and impulsivity, and are often accompanied by hyperactivity. They may be developed in association with an emotional disorder, may induce aggression, stealing, lie, truant, arson, runaway, temper and the like, or may sometimes be accompanied by decrease in cognitive ability and learning ability and decreased social skill.

Methylphenidate is a psychostimulant most often used for the treatment of Attention Deficit Disorder and Attention Deficit Hyperactivity Disorder.

As compared to other psychostimulants, this drug highly frequently expresses good effects and less frequently expresses a bad influence. The effect of methylphenidate for the improvement of attention and behavioral symptoms has been confirmed by many studies.

However, currently available methylphenidate therapeutic agents have many problems. For example, "immediate-release tablets" (for oral administration) of methylphenidate show dispersed blood concentrations of methylphenidate and short half-life of methylphenidate. To ensure appropriate treatment during children's school hours, therefore, the tablets need to be administered frequently at short intervals.

While sustained-release methylphenidate tablets are also commercially available, the reported defects thereof include difficulty in swallowing, insufficient effect of sustained release of methylphenidate, which prevents an appropriate treatment effect during children's school hours, and possible drug abuse.

On the other hand, patch preparation provides many advantages as compared to oral administration preparations, such as convenient administration, improved compliance of patients, easy interruption of administration, absence of first pass effect in the liver, sustained blood concentration for a long time, improved treatment effect and the like. Therefore, a patch preparation is considered to be a superior administration form also for methylphenidate.

For use as a drug for patch preparation, however, methylphenidate is known to be unstable and undergoes degradation in the presence of acid functional groups contained in adhesive, permeation promoter, additive and other components (patent document 1 and the like). Patent document 1 relates to the stability of methylphenidate in an adhesive layer of a patch preparation and discloses a methylphenidate-stabilizing effect of a substituent in an acrylic adhesive. Patent document 1 also discloses a sustained-release effect of methylphenidate in a patch preparation by a combination of an acrylic adhesive and a silicone adhesive as an adhesive.

Generally, the release rate of a drug from a patch preparation is slow as compared to that of preparations for oral administration such as tablet, capsule and the like. Since the release rate is slow, the treatment effect lasts for a long time. Utilizing such characteristics, patch preparation is often used as a sustained-release preparation. However, a patch preparation for transdermal administration of methylphenidate to treat Attention Deficit Disorder or Attention Deficit Hyperactivity Disorder needs to show a certain level of rapid release rate (particularly initial release rate) and superior skin permeability of methylphenidate, because the treatment effect needs to be provided within a comparatively short time (children's school hours). To ensure an appropriate treatment throughout children's school hours, moreover, a therapeutically effective amount needs to be delivered, and a cumulative permeation amount of methylphenidate to the skin needs to be appropriate, i.e., superior drug availability.

[patent document 1]US Patent No. 6,348,211

[Disclosure of the Invention]

[Problems to be Solved by the Invention]

The present invention was made in view of such situation and aims to provide a methylphenidate patch preparation, which shows high stability of a drug (methylphenidate and/or a salt thereof) therein, and superior skin permeability of the drug during use.

In addition, the present invention aims to provide a methylphenidate patch preparation, which shows high stability of a drug (methylphenidate and/or a salt thereof) therein, superior skin permeability of the drug during use and superior drug availability.

[Means of Solving the Problems]

The present inventors have conducted intensive studies in an attempt to solve the above-mentioned problems and found that a patch preparation comprising a support and an adhesive layer formed on at least one surface of the support, wherein the adhesive layer is a plaster comprising methylphenidate and/or a salt thereof as a drug, polyisobutylene as an adhesive, and a liquid plasticizer can afford a methylphenidate patch preparation, which shows less decrease in the drug content, superior skin permeability of the drug and superior drug availability, since methylphenidate and/or a salt thereof are/is stabilized in the plaster (adhesive layer). In addition, they have found that the stability of methylphenidate and/or a salt thereof in the patch preparation can be improved more when the aforementioned liquid plasticizer has an HLB value, which is an index of hydrophilicity-lipophilicity balance, of not more than 3.3, which resulted in the completion of the present invention.

Accordingly, the present invention relates to

(1) a patch preparation comprising a support and an adhesive layer formed on at least one surface of the support, wherein the adhesive layer comprises methylphenidate and/or a salt thereof, polyisobutylene and a liquid plasticizer,
(2) the patch preparation according to (1) above, wherein the liquid plasticizer has an HLB value of 1.0 - 3.3,
(3) the patch preparation according to (1) above, wherein the polyisobutylene comprises a first polyisobutylene having a viscosity average molecular weight of 160,000 - 6,000,000 and a second polyisobutylene having a viscosity average molecular weight of 30,000 - 100,000,
(4) the patch preparation according to (3) above, wherein the ratio of the amount of the first polyisobutylene and the amount of the second polyisobutylene (first polyisobutylene:second polyisobutylene) is 1:0.1 - 10 in weight ratio, and
(5) the patch preparation according to (1) above, wherein the adhesive layer further comprises a tackifier.

[Effect of the Invention]

The present invention can provide a methylphenidate patch preparation, which can stabilize a drug (methylphenidate and/or a salt thereof), particularly minimize a decrease in the drug content during preservation of the preparation, and shows superior skin permeability of the drug during use and superior drug availability. Therefore, the patch preparation of the present invention can be preferably used for the treatment or prophylaxis of Attention Deficit Disorder, Attention Deficit Hyperactivity Disorder and the like. Particularly, it is effective for treating children suffering from the aforementioned disorders through children's school hours and in a comparatively short time, and provides effects such as a sedating effect, decrease of impulsive behaviors, enhanced concentration and the like.

[Best Mode for Carrying out the Invention]

The present invention is explained in detail in the following. The content (amount) indicated in wt% unit of each component constituting the adhesive layer described below shows percentage of the ratio (weight ratio) of each component relative to the total weight of the entire components except the solvent used for forming an adhesive layer, namely, the whole adhesive layer.

The patch preparation of the present invention has a support, and an adhesive layer provided on at least one surface of the support, and is mainly characterized in that the adhesive layer contains methylphenidate as a drug and/or a salt thereof, polyisobutylene as an adhesive, and a liquid plasticizer.

Methylphenidate contains 4 stereoisomers (d-threo, d-erythro, 1-threo and 1-erythro). It preferably contains at least d-threo-methylphenidate, and dl-threoracemate can be used particularly preferably.

In the present invention, examples of the salt of methylphenidate include pharmaceutically acceptable salts such as hydrochloride, hydrobromide, sulfate, phosphate, citrate, lactate, ascorbate, acetate, maleate, tartrate, malate, succinate and the like. In addition, an ester of acid, for example, quaternary ammonium salt of methylphenidate formed by reacting ester of hydrohalic acid such as methyl chloride, methyl bromide, ethyl chloride etc. and the like with methylphenidate can also be mentioned.

Methylphenidate and the salt of methylphenidate may be in the form of solvate such as hydrate and the like. They can be obtained by a known synthesis means.

In the patch preparation of the present invention, while the pharmacological use of methylphenidate and/or a salt thereof (hereinafter to be also referred to as "methylphenidate etc.") is not particularly limited, the patch preparation of the present invention can be particularly preferably used as a therapeutic drug for Attention Deficit Disorder, a therapeutic drug for Attention Deficit Hyperactivity Disorder, a therapeutic drug for narcolepsy and the like.

Either of methylphenidate (free form) or a salt of methylphenidate may be present in the adhesive layer singly or as a mixture thereof. When simply indicated as "methylphenidate" in the present specification, it mean a free form. A free form of methylphenidate is preferable in the present application from the aspect of skin permeability.

While the total content of methylphenidate and/or a salt thereof in the adhesive layer and the concentration thereof in the adhesive layer can be appropriately selected depending on the age, body weight, severity of symptom of patients and the like. Generally, when methylphenidate (dl-threoracemate) is used, the total content thereof in the adhesive layer is preferably not less than 10 mg, more preferably not less than 20 mg, per a patch preparation, from the aspect of delivery of a therapeutically effective amount of a drug. When the total content is too high, an effect corresponding thereto is difficult to obtain. Therefore, the upper limit is preferably not more than 300 mg, more preferably not more than 200 mg, per a patch preparation.

When methylphenidate (dl-threoracemate) is used, the concentration of methylphenidate and/or a salt thereof in the adhesive layer is preferably 1 - 30 wt%, more preferably 5 - 20 wt%, per the total weight of the adhesive layer, from the aspects of the release rate and skin permeability of the drug. When the concentration is less than 1 wt%, a therapeutic or prophylactic effect may not be obtained sufficiently. On the other hand, when it is more than 30 wt%, side effects may be caused due to a high concentration of the drug.

In the present invention, methylphenidate and the like may be delivered to patients by adhering a single patch preparation to patients or adhering plural patch preparations to patients. For convenient treatment, a single patch preparation is preferably adhered.

The adhesion time is preferably at least 6 hr to deliver a therapeutically effective amount of a drug. When the adhesion time is too long, the amount of the drug to be delivered per unit time decreases. Thus, it is preferably not more than 48 hr and more preferably 6 - 24 hr.

The site of application is not particularly limited, either, and the patch preparation can be applied to the skin, mucous membrane (mouth cavity, etc.) and the like. It is generally applied to the skin, for example, arm, abdomen, back, buttock and the like.

While the therapeutically effective amount of methylphenidate and/or a salt thereof varies depending on the age, body weight and severity of symptoms of patients, the kind of the salt of the drug and the like, when methylphenidate (dl-threoracemate) is used, it is, for example, preferably 0.05 - 1.0 mg/kg/day, more preferably 0.075 - 0.3 mg/kg/day, for both children and adults. Monitoring the symptoms of the patients after initial administration, the dose can be appropriately controlled by adjusting the total content of methylphenidate and/or a salt thereof in the adhesive layer (content per sheet of patch preparation), concentration thereof in the adhesive layer, polyisobutylene as the below-mentioned adhesive, the kind and amount of a liquid plasticizer and the like, adhesion time and the like, so as to afford the therapeutically effective amount.

The patch preparation of the present invention has a substantially flat plane form. The flat shape of the patch preparation of the present invention includes, but is not limited to, for example, about rectangle, polygon such as triangle, pentagon and the like, or a shape defined by about straight lines, a shape defined by curved lines such as ellipse, circular shape and the like, a combination thereof and the like. While the size of the patch preparation is not particularly limited, it is preferably a size that can contain the total amount of the above-mentioned drug. The size of the patch preparation can be appropriately selected. For example, when the patch preparation has an about rectangular shape, the length of one side thereof is generally 10 - 100 mm, and the length of other side is generally 10 - 80 mm.

The support to be used in the present invention is not particularly limited and a film or sheet material known per se can be used. A material capable of preventing a liquid plasticizer and a drug from passing through the support and the back face of the patch preparation and getting lost from the back face to lower the content, namely, impermeable to these components, is preferable.

A material having such impermeability is not limited, and examples thereof include polyester such as poly (ethylene terephthalate) and the like, nylon such as nylon-6, nylon-66 and the like, single layer film such as polyvinyl chloride, polyethylene, polypropylene, ethylene-vinyl acetate copolymer, polytetrafluoroethylene, ionomer resin and the like, metal foil, a laminate film thereof and the like.

To improve adhesiveness (anchor property) to the adhesive layer, a laminate film of a non-porous film and a porous film, comprised of the above-mentioned materials, may be used as a support.

A particularly preferable support is a polyester film such as polyethylene terephthalate and the like, from the aspect of low permeability of adhesive layer components such as methylphenidate and the like.

The thickness of the support is preferably 10 - 500 $\mu$m, more preferably 10 - 200 $\mu$m, in consideration of flexibility of the patch preparation and the like. In the case of a thin patch preparation of, for example, a plaster type or an adhesive tape type, it is preferably 10 - 100 $\mu$m. When the support is a laminate film of a non-porous film and a porous film, the thickness of the non-porous film is preferably 5 - 200 $\mu$m, more preferably 5 - 100 $\mu$m, in consideration of suppression of drug permeation and the like.

The adhesive layer formed on at least one surface of the support contains polyisobutylene. Polyisobutylene is considered to have an effect of stabilizing methylphenidate and a salt thereof in the adhesive layer and an effect of promoting permeation of methylphenidate and a salt thereof through the skin. It is preferable as an adhesive in view of the adhesive property of the adhesive layer, safety for the skin, and the balance of stability of methylphenidate and a salt thereof, and preferably used as a component constituting the adhesive layer of the patch preparation of the present invention. A single kind of polyisobutylene may be used or a mixture of two or more kinds of polyisobutylene having different viscosity average molecular weights may be used. From various aspects such as suitable adhesive force and drug solubility and the like, a mixture of two or more kinds of polyisobutylene having different viscosity average molecular weights is preferably used.

In the following, polyisobutylene having cohesiveness necessary for an adhesive layer and added to an adhesive layer as an essential component is referred to as the first polyisobutylene, and further polyisobutylene to be added for various purposes such as increasing adhesive force of the adhesive layer and the like is referred to as the second polyisobutylene.

The first polyisobutylene is not particularly limited, and polyisobutylene having a viscosity average molecular weight of 160,000 - 6,000,000, more preferably 1,000,000 - 5,000,000, most preferably 3,500,000 - 4,500,000, is preferable. When the viscosity average molecular weight is less than 160,000, cohesion strength of the adhesive layer sometimes decreases, possibly causing adhesive residue (residual adhesive layer components on skin surface) when detaching the patch preparation. On the other hand, when it exceeds 6,000,000, polyisobutylene becomes less compatible with other components of the adhesive layer and fails to maintain uniformity of the adhesive layer, again possibly causing adhesive residue when detaching the patch preparation.

When the second polyisobutylene is added for various purposes such as increasing adhesive force of the adhesive layer and the like, polyisobutylene having a viscosity average molecular weight of 30,000 - 100,000, more preferably 40,000 - 80,000, most preferably 50,000 - 60,000, is preferably used. When it is less than 30,000, its amount of addition may be limited, since the cohesion strength of the adhesive layer becomes weak. When it exceeds 100,000, the molecular weight becomes equivalent to or not very different from that of the first polyisobutylene, which may prevent the effect of increasing the adhesive force.

The viscosity average molecular weight in the present invention is determined by calculating a Staudinger index ($J_0$) according to the Schulz-Blaschke equation with the flow time of capillary 1 of Ubbelohde viscometer at 20°C, and with the following formula using the obtained $J_0$ value:

$$J_0 = \eta_{sp}/c(1+0.31\eta_{sp}) \quad \text{(Schulz-Blaschke equation)}$$

$$\eta_{sp} = t/t_0 - 1$$

t: flow time of solution (by Hagenbach-couette correction equation)
to: flow time of solvent (by Hagenbach-couette correction equation)
c: concentration of solution (g/cm$^3$)

$$J_0 = 3.06 \times 10^{-2} Mv^{0.65}$$

Mv: viscosity average molecular weight

The total content (total amount when two or more kinds are used) of polyisobutylene in the adhesive layer is preferably 20 - 85 wt%, more preferably 50 - 85 wt%. When it is less than 20 wt%, the skin adhesion force may be difficult to maintain during adhesion. When it is more than 85 wt%, skin irritation may be developed due to strong skin adhesion force.

When two kinds of polyisobutylene having different viscosity average molecular weights are to be used, the content ratio of the first polyisobutylene and the second polyisobutylene (first polyisobutylene:second polyisobutylene) is preferably 1:0.1 - 10, more preferably 1:0.5 - 5, which affords suitable adhesive force and drug solubility and the like.

The adhesive layer can contain a tackifier when desired. The tackifier may be any appropriately selected from those known in the art of patch and patch preparation. Examples of the tackifier include petroleum resins (e.g., aromatic petroleum resin, aliphatic petroleum resin and the like), terpene resin, rosin resin, coumaroneindene resin, styrene resins (e.g., polystyrene, copolymer of styrene and α-methylstyrene etc.), alicyclic saturated hydrocarbon resins (preferably those having softening point (ring-and-ball method) of 50 - 200°C), hydrogenated petroleum resins (e.g., alicyclic saturated hydrocarbon resin obtained by partial hydrogenation or complete hydrogenation of aromatic petroleum resin etc.), polybutene (e.g., polybutene having kinematic viscosity of 1000 - 10000 mm$^2$/s at 100°C etc.) and the like. Among these, polybutene is preferable, since the preservation stability of methylphenidate and/or a salt thereof becomes fine.

The tackifier may be of one kind, or two or more kinds may be combined. When two or more kinds are to be used in combination, for example, different kinds of resin may be combined, or the same kind of resins having different softening points may be combined.

The content of the tackifier is preferably 15 - 55 wt%, more preferably 20 - 50 wt%. When the content of the tackifier is less than 15 wt%, the tackiness (adhesiveness) and cohesion strength of the adhesive layer sometimes become poor. When it exceeds 55 wt%, the adhesive layer becomes hard and tends to show lower skin adhesiveness.

A liquid plasticizer contained in the adhesive layer can soften the adhesive layer and decrease skin irritation during adhesion and/or detachment of a patch preparation.

As the liquid plasticizer, a plasticizer which is liquid at ambient temperature, shows plasticizing action on an adhesive layer, and compatible with the aforementioned polyisobutylene contained in the adhesive layer can be preferably used. In addition, for example, a plasticizer having an ester group in the structural formula, and a plasticizer that improves transdermal absorbability and preservation stability of methylphenidate and/or a salt thereof are preferable.

Specific examples thereof include fatty acid esters such as fatty acid ester made of a higher fatty acid having a carbon number of 12 to 16 and a lower monovalent alcohol having a carbon number of 1 to 4 and the like; fatty acid having a carbon number of 8 to 10; higher alcohol (preferably higher alcohol having a carbon number of 10 to 30); fats and oils and the like. A liquid plasticizer from among these, which is liquid at ambient temperature, can be used. Specific examples of the liquid plasticizer include squalene and lanolin. In the present specification, being liquid at ambient temperature means showing flowability at 20°C.

Examples of the aforementioned fats and oils include olive oil, castor oil and the like. Preferable examples of the aforementioned fatty acid ester made of a higher fatty acid having a carbon number of 12 to 16 and a lower monovalent alcohol having a carbon number of 1 to 4 include isopropyl palmitate, isopropyl myristate, ethyl laurate and the like. Examples of other preferable fatty acid ester include isotridecyl myristate, ethyl oleate, diisopropyl adipate, octyl palmitate and the like. Preferable examples of the higher alcohol include 2-octyl-1-dodecanol (octyldodecanol).

A liquid plasticizer may be used singly, or may be used in a combination of two or more kinds thereof. The amount of the liquid plasticizer is preferably 5 - 30 wt%, more preferably 10 - 20 wt%. When the amount is less than 5 wt%, the adhesive layer may insufficiently be plasticized and skin irritation sometimes may not be decreased. When it exceeds 30 wt%, the liquid plasticizer sometimes cannot be maintained in the adhesive layer even with the cohesion strength of the adhesive (polyisobutylene). As a result, the liquid plasticizer blooms on the adhesive layer surface and the adhesiveness of the patch preparation to the skin may become inferior.

The liquid plasticizer preferably has an HLB value showing hydrophile-lipophile balance of 1.0 - 3.3. The HLB value ranges from 0 to 20, and a value closer to 0 has a higher lipophilicity and a value closer to 20 has a higher hydrophilicity.

Using a liquid plasticizer having an HLB value of not more than 3.3, the stability of methylphenidate and/or a salt thereof in the patch preparation can be further improved. Since the HLB value of the liquid plasticizer is not less than 1.0, the compatibility of methylphenidate and/or a salt thereof with the adhesive layer can be enhanced, and the possibility of blooming of methylphenidate and/or a salt thereof on the adhesive layer surface can be decreased. The HLB value is more preferably 1.0 - 2.6.

From such aspect, the liquid plasticizer preferably isopropyl palmitate (HLB value 1.62), isopropyl myristate (HLB value 1.82), isotridecyl myristate (HLB value 1.18), ethyl laurate (HLB value 2.14), ethyl oleate (HLB value 1.55), octyl palmitate (HLB value 1.25), 2-ethylhexyl palmitate (HLB value 1.28), 2-octyl-1-dodecanol (octyldodecanol) (HLB value 2.50) and the like.

The HLB value in the present specification is a value calculated according to the following formula by Oda, Teramura et al.

```
HLB value = [(Σinorganic value)/(Σorganic value)]x10
```

Here, the (Σinorganic value) and (Σorganic value) are obtained by respectively adding inorganic values and organic values of the constituent units of molecule of the liquid plasticizer, and the inorganic values and organic values are obtained from the organic concept proposed by Atsushi Fujita (see, for example, Atsushi Fujita, "Chemical Region", Vol. 11, No. 10 (1957), 719-725 etc.). More specifically, the inorganic value is determined by a functional group and, for example, - OH is 100, -COOH is 150, -NH$_2$ (amine) is 70, -COOR (ester group) is 60, -O- is 20, -CO- is 65, aromatic ring (monocycle) is 15, and nonaromatic ring (monocycle) is 10. The organic value is a carbon number of a molecule multiplied by 20. For example, the value for a group having a branched aliphatic group such as isopropyl group is calculated by subtracting 10 from this value, and the like. The detail of the calculation method is also described in the following document and the like besides the above-mentioned document.

Oda, Teramura, "synthesis of surfactant and application thereof", Maki Shoten (1957), page 501

A release liner to protect the adhesive surface can be laminated on the adhesive surface of the adhesive layer of the patch preparation, before applying the patch preparation to the skin. The release liner is not particularly limited, and examples of the material thereof include those known per se in the field. Specific examples thereof include plastic films of polyesters such as poly(ethylene terephthalate), poly(vinyl chloride), poly(vinylidene chloride), various acrylic-based and methacrylic-based polymers, polystyrene, polycarbonate, polyimide, acetyl cellulose, regenerated cellulose (cellophane), celluloid and the like, high-quality paper, glassine paper and the like and a laminate film with polyolefin films and the like. For safety, economic efficiency and drug-transfer properties, a polyester film is preferably used.

The release liner is preferably treated for easy peeling on the interfacial surface side with an adhesive, so as to facilitate peeling from the adhesive layer. While the easy peeling treatment is not limited, a known method can be applied. For example, a treatment for forming a peeling-treated layer using a release agent comprising a curable silicone resin as a main component by a coating method such as bar coating, gravure coating and the like can be applied. The thickness of the peeling-treated layer is preferably 0.01 - 5 $\mu$m in view of ensured release property and uniformity of coated film.

The thickness of the release liner is preferably about 25 - 100 $\mu$m, more preferably about 40 - 80 $\mu$m, so as to maintain the shape of a flexible patch preparation, facilitate holding of a patch preparation and the like.

While the production method of the patch preparation of the present invention mentioned above is not limited, it can be produced, for example, by mixing methylphenidate and/or a salt thereof, polyisobutylene, and components such as a liquid plasticizer, and when desired, a tackifier and the like with a solvent, applying the obtained solution or dispersion to a support, drying same to give a sheet for production of a patch preparation, and cutting the sheet. The aforementioned coating can be performed by, for example, casting, printing or a technique known per se in the art.

While the solvent is not limited, one having compatibility with the aforementioned respective components constituting the adhesive layer, easily volatilizable during a drying process and not impairing the effect of the invention is preferable. Examples of the solvent include aromatic hydrocarbons such as toluene, xylene and the like, aliphatic hydrocarbons such as hexane and the like, esters such as ethyl acetate and the like, alcohols such as ethanol and the like, ethers such as diethyl ether, tetrahydrofuran, etc. and the like. These may be used alone or in a mixture of two or more kinds thereof in combination.

The aforementioned drying may be performed by air-drying, or according to a known method using a dryer, hot air, far-infrared radiation and the like.

While the method of mixing the aforementioned respective components is not limited, examples thereof include kneading machines such as a kneader, a planetary mixer and the like, dispersion machines such as homogenizer and the like, stirring machines such as propeller-type blade stirring machine, etc. and the like. These can be used alone or in a combination of two or more kinds thereof.

The method of the aforementioned cutting is not limited and any known cutting method such as laser, straw cutter and the like can be used.

[Examples]

The present invention is explained in detail in the following by referring to Examples and Comparative Examples, which are not to be construed as limitative. In the following, the parts mean parts by weight.

<Preparation of adhesive compositions A - D>

(Synthetic Example 1)

The first polyisobutylene (viscosity average molecular weight: 4,000,000, 20 parts), the second polyisobutylene (viscosity average molecular weight: 55,000, 20 parts), and polybutene (kinetic viscosity: 4000 mm$^2$/s (100°C), 60 parts) as a tackifier were mixed to give an adhesive composition A containing polyisobutylene.

(Synthetic Example 2)

Under an inert gas atmosphere, 2-ethylhexyl acrylate (71 parts), vinyl acetate (22 parts) and 2-hydroxyethyl acrylate (7 parts) were subjected to solution polymerization in ethyl acetate at 60°C to give an acrylic copolymer adhesive composition B.

(Synthetic Example 3)

Under an inert gas atmosphere, 2-ethylhexyl acrylate (95 parts) and acrylic acid (5 parts) were subjected to solution polymerization in ethyl acetate at 60°C to give an acrylic copolymer adhesive composition C.

(Synthetic Example 4)

Under an inert gas atmosphere, 2-ethylhexyl acrylate (75 parts), N-vinyl-2-pyrrolidone (22 parts) and acrylic acid (3 parts) were subjected to solution polymerization in ethyl acetate at 60°C to give an acrylic copolymer adhesive composition D.

<Production of patch preparation>

(Examples 1 - 4)

Using adhesive composition A (3.3 g) and toluene (6.3 mL) as a solvent and according to the blending ratios shown in Table 1, viscous toluene solutions of adhesive composition were prepared, and the obtained solutions were applied to a polyethylene terephthalate (PET) release liner (thickness 75 μm) subjected to a silicone release treatment, such that the thickness of the adhesive layer after drying was 100 μm, and dried in a hot-air circulation type dryer at 80°C for 10 min to form an adhesive layer. The adhesive surface of the adhesive layer was adhered to a 25 μm-thick polyethylene terephthalate (PET) support to give a sheet for production of a patch preparation. This was cut with a straw cutter to give a patch preparation sheet (100 mmx400 mm). One patch preparation contained about 40 mg of methylphenidate.

(Comparative Example 1)

In the same manner as in Examples 1 - 4 except that a viscous ethyl acetate solution of an adhesive composition was prepared using adhesive composition B and according to the blending ratio shown in Table 1, and an aging treatment at 60°C was performed for 48 hr after drying in a hot-air circulation type dryer, a patch preparation sheet was obtained.

(Comparative Example 2)

In the same manner as in Examples 1 - 4 except that a viscous ethyl acetate solution of an adhesive composition was prepared using adhesive composition C and according to the blending ratio shown in Table 1, and an aging treatment at 60°C was performed for 48 hr after drying in a hot-air circulation type dryer, a patch preparation sheet was obtained. Note that AL-A (aluminum acetylacetonate) is a crosslinking agent.

(Comparative Example 3)

In the same manner as in Examples 1 - 4 except that a viscous ethyl acetate solution of an adhesive composition was prepared using adhesive composition D and according to the blending ratio shown in Table 1, and an aging treatment at 60°C was performed for 48 hr after drying in a hot-air circulation type dryer, a patch preparation sheet was obtained. Note that AL-A (aluminum acetylacetonate) is a crosslinking agent.

[Table 1]

| blending ratio (unit: part) | | | | |
|---|---|---|---|---|
| | adhesive composition | MPH | liquid plasticizer | AL-A |
| Example 1 | adhesive composition A82 | 8 | isopropyl palmitate 10 | - |
| Example 2 | adhesive composition A82 | 8 | 2-octyl-1-dodecanol 10 | - |
| Example 3 | adhesive composition A82 | 8 | oleic acid 10 | - |
| Example 4 | adhesive composition A82 | 8 | dipropylene glycol 10 | - |
| Comparative Example 1 | adhesive composition B82 | 8 | isopropyl palmitate 10 | - |
| Comparative Example 2 | adhesive composition C82 | 8 | isopropyl palmitate 10 | 0.01 |

(continued)

| blending ratio (unit: part) | | | | |
|---|---|---|---|---|
| | adhesive composition | MPH | liquid plasticizer | AL-A |
| Comparative Example 3 | adhesive composition D82 | 8 | isopropyl palmitate 10 | 0.01 |

Note 1) MPH means methylphenidate (dl-threoracemate).
2) AL-A is aluminum acetylacetonate.
3) HLB value of each liquid plasticizer is as follows. isopropyl palmitate: 1.62, 2-octyl-1-dodecanol (octyldodecanol): 2.50, oleic acid: 4.17, dipropylene glycol: 18.33

Experimental Example 1 (content stability test)

The patch preparations obtained in Example 1 and Comparative Examples 1 - 3 were preserved under the preservation conditions at 60°C for 2 weeks, methylphenidate in a plaster (adhesive layer) was quantified by HPLC before the start of preservation and after 2 weeks' preservation, and the ratio of methylphenidate remaining after the preservation was obtained. The HPLC conditions were as follows. For each of Example 1 and Comparative Examples 1 - 3, methylphenidate was quantified at 3 points of the plaster (adhesive layer) before the start of preservation and after 2 weeks' preservation, the ratio of methylphenidate remaining at 3 points was obtained from the quantification values, and the average value and standard deviation thereof were determined. The ratio of remaining methylphenidate is expressed as percentages (wt%) of methylphenidate after 2 weeks' preservation relative to that before the start of preservation. The average value and standard deviation thereof are shown in Table 2.

(HPLC conditions)

detector: ultraviolet absorption spectrophotometer (detection wavelength 220 nm)
column: Inertsil ODS-3 (GL Sciences, Inc.)
column temperature: 25°C
mobile phase: water/acetonitrile/triethylamine mixture (volume ratio 160:40:1) added with phosphoric acid to adjust the mixture to pH 3.0.
flow: controlled such that retention time of methylphenidate was about 6 min.

[Table 2]

| residual rate of methylphenidate (wt%) | | |
|---|---|---|
| | average value | standard deviation |
| Example 1 | 100.3 | 1.44 |
| Comparative Example 1 | 88.0 | 0.19 |
| Comparative Example 2 | 11.6 | 0.82 |
| Comparative Example 3 | 23.2 | 0.64 |

As is clear from Table 2, methylphenidate content did not decrease even under harsh preservation conditions at 60°C in Example 1. However, the content decreased in Comparative Examples 1 - 3 and, particularly in Comparative Example 2, the ratio (average value) of residual methylphenidate was 11.6 wt%, showing a remarkable decrease in the content.

Experimental Example 2 (human skin permeability test)

Using the patch preparations obtained in Example 1 and Comparative Examples 1 - 3, human skin permeability test was performed. In the test, a patch preparation (cut into 6 mmɸ circle) was adhered to the surface of a stratum corneum layer of the isolated human skin (cut into 16 mmɸ circle, thickness 20 µm), set in a glass diffusion cell (e.g.,

Franz cell available from Keystone Scientific K.K. and the like), such that the dermis layer side of the skin came into contact with a receptor solution, sampling the receptor solution at given time intervals, and quantifying methylphenidate in the receptor solution by HPLC. Saline at 32°C was used as a receptor solution. The HPLC conditions were the same as in Experimental Example 1, except the detection wavelength at 257 nm.

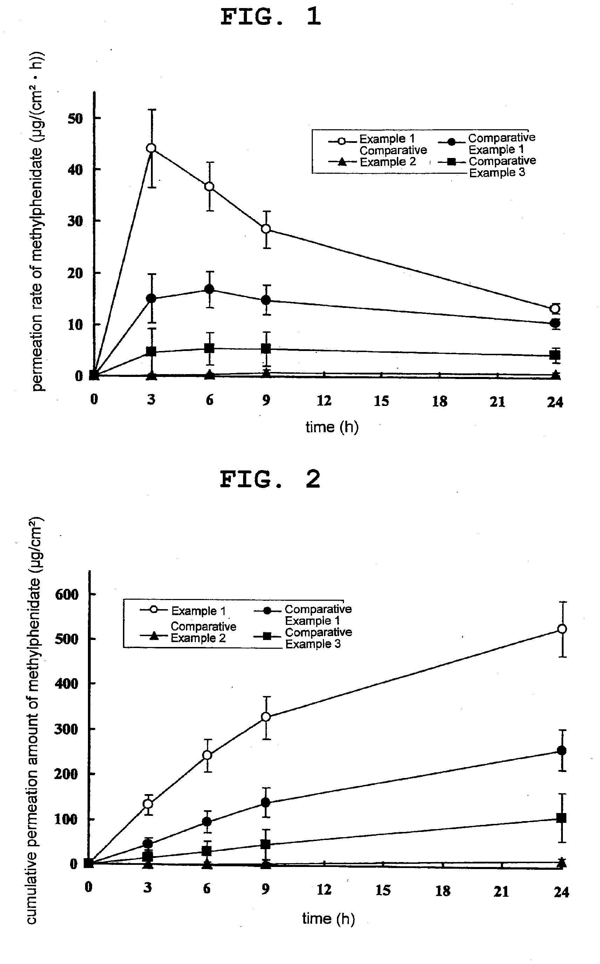

The test was performed 5 times for each of the patch preparations obtained in Example 1 and Comparative Examples 1 - 3. The permeation rate of methylphenidate through the skin as determined from the quantified value is shown in Fig. 1, cumulative permeation amount of methylphenidate through the skin is shown in Fig. 2, and permeability evaluation parameters are shown in Table 3. In the Table, each plot shows an average value of 5 tests and the bar shows standard deviation.

[Table 3]

| permeability evaluation parameters | | | |
|---|---|---|---|
| | maximum skin permeation rate ($\mu$g/ (cm$^2$ h)) | cumulative permeation amount ($\mu$g/cm$^2$) | drug availability (%) |
| Example 1 | 44.2 | 532 | 66.4 |
| Comparative Example 1 | 16.8 | 263 | 35.5 |
| Comparative Example 2 | 0.8 | 16 | 3.8 |
| Comparative Example 3 | 5.4 | 113 | 16.4 |
| Note 1) Cumulative permeation amount is an average after 24 hr. 2) Drug availability is a ratio of the cumulative permeation amount ($\mu$g/cm$^2$) after 24 hr relative to methylphenidate content ($\mu$g/cm$^2$) of patch preparation in percentage (wt%). | | | |

As is clear from Table 3, Fig. 1 and Fig. 2, the permeation rate and cumulative permeation amount of methylphenidate through the skin were high in Example 1, and methylphenidate availability was 66.4 wt%. In contrast, the permeation rate and cumulative permeation amount of methylphenidate through the skin were low in Comparative Examples 1 - 3, and methylphenidate availability was not more than 40 wt%.

Experimental Example 3 (stability testing of the drug content)

The patch preparations obtained in Examples 1 - 4 were subjected to a test in the same manner as in Experimental Example 1. The ratio of methylphenidate remaining after preservation was determined, and the average value and standard deviation shown in Table 4 were obtained.

[Table 4]

| ratio of residual methylphenidate (wt%) | | |
|---|---|---|
| | average value | standard deviation |
| Example 1 | 99.7 | 0.84 |
| Example 2 | 90.4 | 0.65 |
| Example 3 | 15.2 | 0.16 |
| Example 4 | 21.9 | 1.00 |

As is clear from Table 4, the methylphenidate content did not decrease even under harsh preservation conditions at 60°C in Example 1. However, the content decreased in Examples 3 and 4 and, particularly in Example 3, the ratio (average value) of residual methylphenidate was 15.2 wt%, showing a remarkable decrease in the content.

[Industrial Applicability]

Since the patch preparation of the present invention shows superior skin permeability, and is superior in methylphenidate availability, it can be preferably used for the treatment of Attention Deficit Disorder, Attention Deficit Hyperactivity Disorder and the like. Particularly, it is effective for treating children suffering from the aforementioned disorders through children's school hours and in a comparatively short time.

[Brief Description of the Drawings]

Fig. 1 is a graph showing time-course changes of permeation rate of methylphenidate through the skin in Experimental Example 2.
Fig. 2 is a graph showing time-course changes of cumulative permeation amount of methylphenidate through the skin in Experimental Example 2.

**Claims**

1. A patch preparation comprising a support and an adhesive layer formed on at least one surface of the support, wherein the adhesive layer comprises methylphenidate and/or a salt thereof, polyisobutylene and a liquid plasticizer.

2. The patch preparation according to claim 1, wherein the liquid plasticizer has an HLB value of 1.0 - 3.3.

3. The patch preparation according to claim 1, wherein the polyisobutylene comprises a first polyisobutylene having a viscosity average molecular weight of 160,000 - 6,000,000 and a second polyisobutylene having a viscosity average molecular weight of 30,000 - 100,000.

4. The patch preparation according to claim 3, wherein the ratio of the amount of the first polyisobutylene and the amount of the second polyisobutylene (first polyisobutylene:second polyisobutylene) is 1:0.1 - 10 in weight ratio.

5. The patch preparation according to claim 1, wherein the adhesive layer further comprises a tackifier.

# FIG. 1

# FIG. 2

Europäisches
Patentamt

European
Patent Office

Office européen
des brevets

# EUROPEAN SEARCH REPORT

Application Number

EP 10 15 3194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/102291 A1 (MANTELLE JUAN [US] ET AL) 1 August 2002 (2002-08-01) | 1,2,5 | INV.<br>A61K9/70<br>A61K31/4458 |
| Y | * claim 1 *<br>* paragraphs [0027], [0041] *<br>* example 1 *<br>----- | 3,4 | |
| Y | EP 2 110 125 A1 (NITTO DENKO CORP [JP]) 21 October 2009 (2009-10-21)<br>* paragraphs [0015] - [0018], [0058] - [0060] *<br>----- | 3,4 | |

TECHNICAL FIELDS
SEARCHED      (IPC)

A61K

The present search report has been drawn up for all claims

| Place of search | Date of completion of the search | Examiner |
|---|---|---|
| Munich | 12 July 2010 | Vázquez Lantes, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 15 3194

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2010

| Patent document cited in search report | | Publication date | Patent family member(s) | | Publication date |
|---|---|---|---|---|---|
| US 2002102291 | A1 | 01-08-2002 | NONE | | |
| EP 2110125 | A1 | 21-10-2009 | CA | 2662499 A1 | 16-10-2009 |
| | | | CN | 101574332 A | 11-11-2009 |
| | | | JP | 2009275040 A | 26-11-2009 |
| | | | US | 2009264806 A1 | 22-10-2009 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6348211 B

**Non-patent literature cited in the description**

- **ATSUSHI FUJITA.** *Chemical Region,* 1957, vol. 11 (10), 719-725

- **ODA ; TERAMURA.** synthesis of surfactant and application thereof. Maki Shoten, 1957, 501